# EUROPEAN PATENT SPECIFICATION

(11) **EP 3 243 431 B1**
(45) Date of publication and mention of the grant of the patent: **28.04.2021**
(21) Application number: 17179049.6
(22) Date of filing: 11.06.2014
(51) Int. Cl.: G04B 37/12, G04G 21/02

(54) **A STRAP FOR A PORTABLE PULSE MEASURING DEVICE AND A PORTABLE PULSE MEASURING DEVICE**
GURT FÜR EINE TRAGBARE PULSMESSUNGSVORRICHTUNG UND TRAGBARE PULSMESSUNGSVORRICHTUNG
SANGLE POUR DISPOSITIF PORTABLE DE MESURE D'IMPULSION, ET DISPOSITIF PORTABLE DE MESURE D'IMPULSION

(30) Priority: 11.06.2013 FI 20135640
(43) Date of publication of application: 15.11.2017
(62) Divisional of application: 14811340.0
(73) Proprietor: PulseOn Oy, 02150 Espoo (FI)
(72) Inventor: Nousiainen, Jari, 02780 Espoo (FI); Korhonen, Ilkka, 37560 Lempäälä (FI); Saunamäki, Jarkko, 01200 Vantaa (FI); Hattula, Jaakko, 02130 Espoo (FI)
(74) Representative: Papula Oy

(56) References cited:
- EP-A1- 1 552 993
- EP-A1- 2 555 676
- US-A- 5 485 848
- US-A1- 2002 151 775
- US-A1- 2008 146 980
- US-A1- 2008 312 682
- US-A1- 2008 312 682
- US-A1- 2009 168 612
- US-A1- 2010 146 683

## Description

### FIELD OF THE INVENTION

The invention relates to a portable pulse measuring device comprising a strap.

### BACKGROUND OF THE INVENTION

Pulse can be measured using, for example, portable pulse measuring devices. A portable pulse measuring device may measure pulse optically. Another possibility is to use a separate heart rate belt around one's chest, and the belt then transmits measured pulse signals wirelessly to a monitoring device, for example, attached on a wrist or to an application running in a mobile phone.

The optical pulse measurement is based on the fact that light is emitted by a light source towards body tissue and at least one detector is configured to detect the intensity of reflected light after propagation through the human body tissue. There are several challenges when measuring pulse optically. The optical measurement is based on light absorption changes caused by blood flow in a lighted area. If the shape of the lighted area changes during the measurement, for example, due to movement of the pulse measuring device, the measurement is disturbed. Thus, for example, movements of a hand and of a human cause errors to the measurement in many ways. In order to avoid problems in the measurement, the portable pulse device needs to be as stable as possible in relation to skin and needs to minimize mechanical changes in tissue area during movement.

There are many ways to address the above problems. One solution is to tighten a strap of pulse a measuring device more. The problem, however, is that a user may tighten the strap too much, which in turn is uncomfortable and prevents blood flow in tissue. In turn, too loose tightening of the strap allows the portable pulse measuring device to move too much in relation, for example, to a wrist and body tissue.

It is possible to solve the above problems by design, for example, by making the device light, by optimizing the friction between skin and the device by material selection, or by making the device or strap of the device wider. Although these aspects may alleviate the problems in some cases, the basic problem still remains - how to provide an optimal strap tightening for a strap of a pulse measuring device.

US 2008/312682 A1 discloses a device for indicating the pressure applied to an object by a strap.

US 5 485 848 A discloses a non-invasive, non-intrusive, convenient and portable device for monitoring a user's arterial blood pressure is provided

US 2009/168612 A1 discloses a portable pulse measuring device comprising a mechanical indicator configured to indicate tightness of a strap, wherein the indicator forms part of the strap.

US 2008/0146980 A1 discloses a portable device for applying intermittent pressure to a limb comprising a mechanical indicator configured to indicate tightness of a strap, wherein the indicator forms part of the main body but not a linking part thereof.

### SUMMARY OF THE INVENTION

According to the present invention, a portable pulse measuring device in accordance with claims 1 and 2 is provided.

According to a first aspect, there is provided a portable pulse measuring device comprising a main body comprising a link part, wherein the link part is an integral part of the main body, a strap configured to fasten the portable pulse measuring device on a human, and wherein the strap is connected to the link part and wherein the link part comprises a window in which a mechanical indicator configured to indicate tightness of the strap is movable to indicate the tightness of the strap. The link part comprises a rotating part to which the strap is attached, and wherein the indicator is attached to the rotating part such that when the rotating part rotates, the indicator moves in the window.

According to a second aspect, there is provided a portable pulse measuring device comprising a main body comprising a link part, wherein the link part is an integral part of the main body, a strap configured to fasten the portable pulse measuring device on a human, and wherein the strap is connected to the link part and wherein the link part comprises a window in which a mechanical indicator configured to indicate tightness of the strap is movable to indicate the tightness of the strap. The link part comprises a sliding part to which the strap is attached, and wherein the indicator is attached to the sliding part such that when the sliding part moves, the indicator moves in the window.

### BRIEF DESCRIPTION OF THE DRAWINGS

The accompanying drawings, which are included to provide a further understanding of the invention and constitute a part of this specification, illustrate embodiments of the invention and together with the description help to explain the principles of the invention. In the drawings:
**Figure 1A** discloses an arrangement comprising a mechanical indicator configured to indicate tightness of a strap of a portable pulse measuring device according to one embodiment of the invention.
**Figure 1B** discloses a cross-section view of the arrangement of Figure 1A.
**Figure 2** discloses an arrangement comprising a mechanical indicator configured to indicate tightness of a strap of a portable pulse measuring device according to one embodiment of the invention.

### DETAILED DESCRIPTION

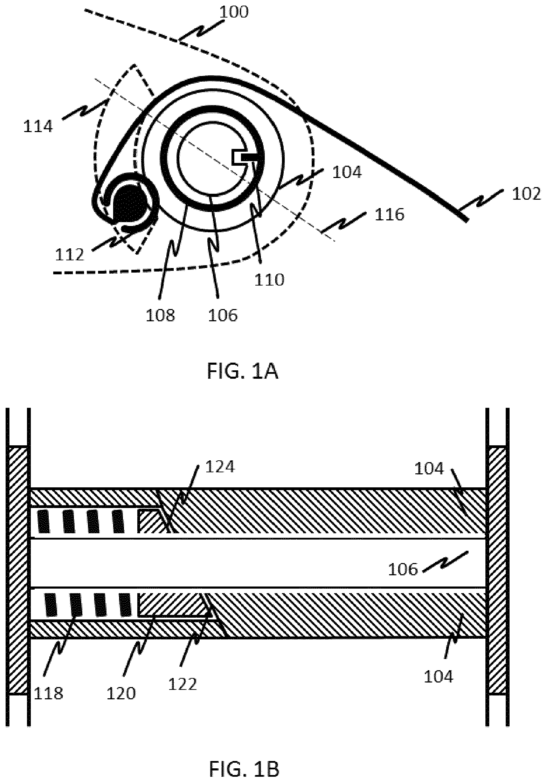

Figure 1A discloses an arrangement comprising a mechanical indicator configured to indicate tightness of a strap 102 of a portable pulse measuring device according to one embodiment of the invention.

A link part 100 of the portable pulse measuring device provides an attachment point to a strap 102. The link part 100 may be removably attachable to a main body (not shown) of the portable pulse measuring device, or alternatively, the link part 100 may be an integral part of the main body. The link part 100 includes a fixed axle 106. A sliding part and a resilient member, for example a spring 108, are arranged around the fixed axle 104. Instead of the spring, any other resilient member may be used. A rotation blocker 110 is attached to the sliding part 108 in order to prevent the sliding part 108 to rotate around the fixed axle 106. A rotating non-sliding part 104 is arranged as an outmost element and the strap 102 is in contact with the rotating non-sliding part 104 in a section of its circumference, as illustrated in Figure 1A. Reference number 112 indicates that the strap 102 is attached to the rotating non-sliding part 104. The link part 100 includes a window 114, for example a hole, in which an indicator is movable and the location of the indicator in the hole 114 depends on the tightness of the strap 102 when a user of the portable pulse measuring device fastens the device, for example, around his wrist.

In one embodiment, the indicator is attached to the rotating non-sliding part 104 and thus it moves when the rotating non-sliding part 104 rotates. The indicator may be a peg which moves in the window 114. In another embodiment, the indicator is a plate movable in the window 114 and it comprises a scale. A pointer has been arranged in the link part 100. When the plate moves as a result of pulling the strap, the pointer points to a certain point in the scale on the plate.

As illustrated in Figure 1A, the hole 114 may take a form of a slot which is radially arranged in relation to the fixed axle 106. Reference number 116 refers to a cross-section which is illustrated in more detail in Figure 1B.

In one embodiment, the window 114 is a hole in the link part. In another embodiment, the link part 100 comprises transparent section through which the indicator can be seen. For example, the link part 100 may be partly or wholly made of plastic and it may be partly or wholly transparent.

Figure 1B discloses a cross-section view of the arrangement of Figure 1A. As illustrated in Figure 1B the rotating non-sliding part 104 partially directly surrounds the fixed axle 106 and is arranged to be rotatable about the fixed axle 106. The remaining part of the fixed axle 106 not directly surrounded by the rotating non-sliding part 104 is occupied by a spring 118 and a non-rotating sliding part 120.

When the strap 102 is pulled the rotating non-sliding part 104 rotates around the fixed axle 106. An inclined surface 122 of the rotating non-sliding part 104 faces towards an inclined surface 124 of the sliding non-rotating part 120. When the rotating non-sliding part 104 rotates it presses the sliding non-rotating part 120, and due to the inclined surfaces 122 and 124, the sliding non-rotating part 120 moves and compresses the spring 118. Although not illustrated in Figure 1B, this action also moves the indicator in the hole 114. When the pulling stops and the tightness of the strap 102 is reduced, the spring 118 presses the sliding non-rotating part 120 and the sliding non-rotating part 120 generates a rotating force for the rotating non-sliding part 104 and thus the rotating non-sliding part 104 turns to its relaxed position.

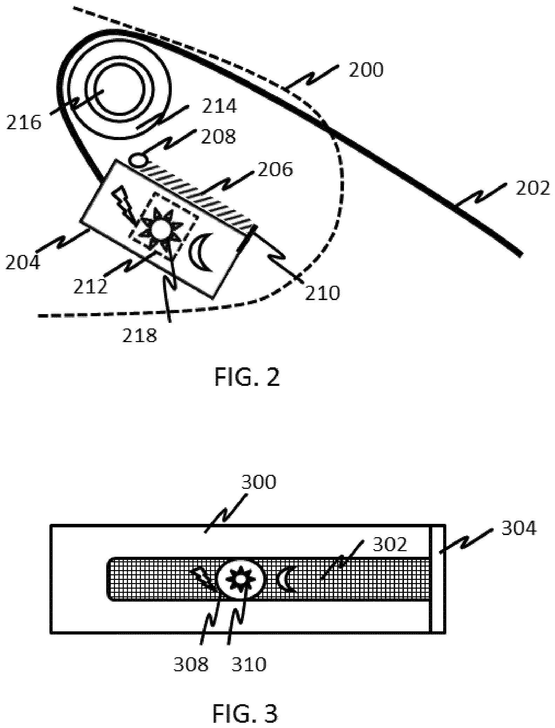

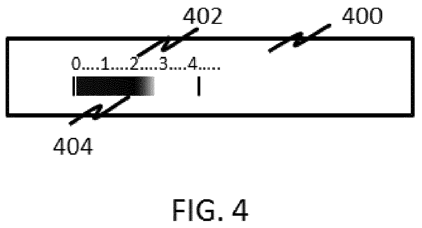

Figure 2 discloses an arrangement comprising a mechanical indicator configured to indicate tightness of a strap 202 of a portable pulse measuring device according to one embodiment of the invention. Whereas Figure 1A disclosed a turning force indicator Figure 2 discloses a sliding force indicator.

A link part 200 of the portable pulse measuring device provides an attachment point to the strap 202. The link part 200 may be removably attachable to a main body (not shown) of the portable pulse measuring device or alternatively the link part 200 may be an integral part of the main body. The link part 200 includes a fixed axle 216. A rotating part 214 is arranged to be rotatably attached to the fixed axle 216. The strap 202 is arranged to be partially in contact with the rotating part 214 on the circumference of the rotating part 214, as disclosed in Figure 2. A sliding part 204 is arranged in the link part 200 and the strap 202 is attached to the sliding part 204.

A first spring blocker 208 is arranged in the link part 200 and a second spring blocker is arranged in the sliding part 204. A spring 206 is arranged between the first spring blocker 208 and the second spring blocker 210. In one embodiment, the sliding part 204 comprises guiders which keep the sliding part 204 on its sliding track in the link part 200. When the strap 202 is pulled, the sliding part 204 moves and the spring 206 compresses. Instead of the spring 206, any other resilient member may be used.

The link part 200 also includes a window 212 through which an indicator 218 in the sliding part 204 or attached to the sliding part 204 can be seen. In one embodiment, the window 212 is a hole in the link part 200. In another embodiment, the link part 200 comprises a transparent section through which the indicator in the sliding part 204 or attached to the sliding part 204 can be seen. For example, the link part 200 may be partly or wholly made of plastic. Furthermore, it may be partly or wholly transparent.

As an example of the indicator, Figure 2 discloses that the sliding part includes three patterns for indicating the tightness of the strap 202. Only one pattern can be seen in whole at a time through the window 212 in the link part 200. It is evident that Figure 2 discloses only one example of a possible indicator. In another embodiment, an elongated slot may be arranged in the link part 200 and an indicator attached to the sliding part 204 moves in the elongated slot and indicates the current tightness of the strap 202.

A benefit of the embodiments disclosed in Figures 1A, 1B and 2 is that the guided adjustment of tightness of the strap enables the use of the portable pulse measuring device in various operating situations. Moreover, the solution enables optimal strap tightness and avoids excessive loosening or tightening. Furthermore, the disclosed solution also takes into account physiological variations between individuals. Furthermore, the embodiments disclosed in Figures 1A, 1B and 2 are also advantageous when measuring pulse with pulse measuring devices that use optical pulse measurement techniques since undesired movements of the device may cause disturbances in the measurements. With the disclosed embodiments it is possible to ensure optimal strap tightness of the strap.

It is obvious to a person skilled in the art that with the advancement of technology, the basic idea of the invention may be implemented in various ways. The invention and its embodiments are thus not limited to the examples described above, instead they may vary within the scope of the claims.

## Claims

1. A portable pulse measuring device comprising:
a main body comprising a link part (100) ;
a strap (102) configured to fasten the portable pulse measuring device on a human; and
wherein the strap (102) is connected to the link part (100) and wherein the link part (100) comprises a window (114) in which a mechanical indicator configured to indicate tightness of the strap (102) is movable to indicate the tightness of the strap (102);
**characterized in that** the link part (100) is an integral part of the main body, and
the link part (100) comprises a rotating part (104) to which the strap (102) is attached, wherein the indicator is attached to the rotating part (104) such that when the rotating part (104) rotates, the indicator moves in the window (114).

2. A portable pulse measuring device comprising:
a main body comprising a link part (100) ;
a strap (102) configured to fasten the portable pulse measuring device on a human; and
wherein the strap (102) is connected to the link part (100) and wherein the link part (100) comprises a window (114) in which a mechanical indicator configured to indicate tightness of the strap (102) is movable to indicate the tightness of the strap (102);
**characterized in that**
the link part (100) is an integral part of the main body, and
the link part (100) comprises a sliding part (120) to which the strap (102) is attached, wherein the indicator is attached to the sliding part (120) such that when the sliding part (120) moves, the indicator moves in the window (114).

## Patentansprüche

1. Tragbares Pulsmessgerät, das Folgendes aufweist:
einen Hauptkörper, der ein Verbindungsteil (100) aufweist;
ein Gurt (102), der ausgestaltet ist, das tragbare Pulsmessgerät an einem Menschen zu befestigen; und
wobei der Gurt (102) mit dem Verbindungsteil (100) verbunden ist und wobei das Verbindungsteil (100) ein Fenster (114) aufweist, in dem ein mechanischer Indikator, der so ausgestaltet ist, dass er die Straffheit des Gurtes (102) anzeigt, beweglich ist, um die Straffheit des Gurtes (102) anzuzeigen;
**dadurch gekennzeichnet, dass**
das Verbindungsteil (100) ein integraler Teil des Hauptkörpers ist, und
das Verbindungsteil (100) ein rotierendes Teil (104) aufweist, an dem der Gurt (102) befestigt ist, wobei der Indikator an dem rotierenden Teil (104) derart befestigt ist, dass, wenn sich das rotierende Teil (104) dreht, sich der Indikator in dem Fenster (114) bewegt.

2. Tragbares Pulsmessgerät, das Folgendes aufweist:
einen Hauptkörper, der ein Verbindungsteil (100) aufweist;
ein Gurt (102), der ausgestaltet ist, das tragbare Pulsmessgerät an einem Menschen zu befestigen; und
wobei der Gurt (102) mit dem Verbindungsteil (100) verbunden ist und wobei das Verbindungsteil (100) ein Fenster (114) aufweist, in dem ein mechanischer Indikator, der so ausgestaltet ist, dass er die Straffheit des Gurtes (102) anzeigt, beweglich ist, um die Straffheit des Gurtes (102) anzuzeigen;
**dadurch gekennzeichnet, dass**
das Verbindungsteil (100) ein integraler Teil des Hauptkörpers ist, und
das Verbindungsteil (100) ein gleitendes Teil (120) aufweist, an dem der Gurt (102) befestigt ist, wobei der Indikator an dem gleitenden Teil (120) derart befestigt ist, dass, wenn sich das gleitende Teil (120) bewegt, sich der Indikator in dem Fenster (114) bewegt.

## Revendications

1. Dispositif portable de mesure de pouls, comprenant:
un corps principal comprenant une partie de liaison (100);
une sangle (102) configurée pour fixer le dispositif portable de mesure de pouls à un être humain; et
dans lequel la sangle (102) est connectée à la partie de liaison (100) et dans lequel la partie de liaison (100) comprend une fenêtre (114) dans laquelle un indicateur mécanique configuré pour indiquer le serrement de la sangle (102) peut se déplacer pour indiquer le serrement de la sangle (102);
**caractérisé par le fait que**
la partie de liaison (100) fait partie intégrante du corps principal, et
la partie de liaison (100) comprend une partie rotative (104) à laquelle est fixée la sangle (102), où l'indicateur est fixé à la partie rotative (104) de sorte que, lorsque la partie rotative (104) tourne, l'indicateur se déplace dans la fenêtre (114).

2. Dispositif portable de mesure de pouls, comprenant:
un corps principal comprenant une partie de liaison (100);
une sangle (102) configurée pour fixer le dispositif portable de mesure de pouls à un être humain; et
dans lequel la sangle (102) est connectée à la partie de liaison (100) et dans lequel la partie de liaison (100) comprend une fenêtre (114) dans laquelle un indicateur mécanique configuré pour indiquer le serrement de la sangle (102) peut se déplacer pour indiquer le serrement de la sangle (102);
**caractérisé par le fait que**
la partie de liaison (100) fait partie intégrante du corps principal, et
la partie de liaison (100) comprend une partie coulissante (120) à laquelle est fixée la sangle (102), où l'indicateur est fixé à la partie coulissante (120) de sorte que, lorsque la partie coulissante (120) se déplace, l'indicateur se déplace dans la fenêtre (114).
